# EUROPÄISCHE PATENTANMELDUNG

(11) **EP 4 744 705 A1**
(43) Veröffentlichungstag der Anmeldung: **20.05.2026**
(21) Anmeldenummer: 25213196.6
(22) Anmeldetag: 04.11.2025
(51) Int. Cl.: A61M 16/16, A61M 16/10

(54) **ATEMGASBEFEUCHTER FÜR EIN BEATMUNGSGERÄT**

(30) Priorität: 14.11.2024 DE 102024133290
(71) Anmelder: Löwenstein Medical Technology S.A., 2557 Luxembourg (LU)
(72) Erfinder: Gardein, Joachim, 38430 Icod de los Vinos (ES); Hein, Stefan, 22529 Hamburg (DE)
(74) Vertreter: Löwenstein Medical IP

(57) **Zusammenfassung**

Die Erfindung betrifft einen Atemgasbefeuchter (1) mit einem Gehäuse (20) umfassend ein Gehäuseoberteil (21) und ein Gehäuseunterteil (23) mit einer Wasserkammer (5) zum Befeuchten und/oder Erwärmen eines Atemgases, wobei der Atemgasbefeuchter (1) einen Atemgaseinlass (2) und einen Atemgasauslass (4) umfasst, wobei zwischen dem Atemgaseinlass (2) und dem Atemgasauslass (4) ein Atemgaspfad (3) zum Leiten des Atemgases vom Atemgaseinlass (2) zum Atemgasauslass (4) ausgebildet ist, der durch die Wasserkammer (5) geführt ist, wobei der Atemgaspfad (3) einen wasserdichten Kanal (8) zum Leiten des befeuchteten und/oder angewärmten Atemgases umfasst, der an einem Ende (7) in die Wasserkammer (5) mündet und an einem anderen Ende in den Atemgasauslass (4) mündet, wobei der Kanal (8) derart ausgebildet ist, dass der Atemgasauslass (4) am Gehäuseunterteil (23) angeordnet und/oder zumindest teilweise durch dieses ausgebildet ist.

## Beschreibung

### Technisches Gebiet

Die Erfindung betrifft einen Atemgasbefeuchter zum Befeuchten und/oder Erwärmen eines Atemgases. Des Weiteren betrifft die Erfindung ein Beatmungsgerät mit einem solchen Atemgasbefeuchter.

### Stand der Technik

Ein Atemgasbefeuchter ist eine Vorrichtung zum Befeuchten und/oder Erwärmen eines Atemgases. Patienten, die künstlich beatmet oder in ihrer Atmung unterstützt werden, können die Beatmung mit zu kalter oder zu trockener Luft als unangenehm empfinden. Zudem kann eine künstliche Beatmung zu trockenen Atemwegen führen. Daher ist es von Vorteil, wenn das einzuatmende Atemgas mithilfe eines Atemgasbefeuchters befeuchtet und erwärmt wird. Somit kann eine Austrocknung der Schleimhäute abgemildert oder vermieden und die Akzeptanz der Beatmung oder Atemunterstützung seitens des Patienten gesteigert werden.

Atemgasbefeuchter weisen ein Flüssigkeitsdepot auf, um das Atemgas über einen längeren Zeitraum befeuchten zu können. Um einen ungewollten oder unkontrollierten Flüssigkeitsaustritt aus dem Gerät zu verhindern, sollte das Gerät eine gewisse Standfestigkeit aufweisen und zudem derart eingerichtet sein, dass die Flüssigkeit auch bei einer Neigung des Gerätes nicht unkontrolliert aus dem Gerät austreten kann.

### Offenbarung der Erfindung

Eine Aufgabe der Erfindung kann darin gesehen werden, einen verbesserten Atemgasbefeuchter zum Befeuchten und/oder Erwärmen eines Atemgases zu schaffen. Eine weitere Aufgabe der Erfindung kann darin gesehen werden, ein verbessertes Beatmungsgerät mit Atemgasbefeuchter bereitzustellen.

Diese Aufgabe wird durch die Gegenstände der unabhängigen Ansprüche gelöst. Vorteilhafte Ausführungsformen der Erfindung sind in den abhängigen Ansprüchen, der nachfolgenden Beschreibung und den beigefügten Figuren dargelegt.

Ein erster Aspekt der Erfindung betrifft einen Atemgasbefeuchter zum Befeuchten und/oder Erwärmen eines Atemgases, insbesondere zur Verwendung mit einem Beatmungsgerät. Der Atemgasbefeuchter umfasst ein Gehäuse mit einem Gehäuseoberteil und einem Gehäuseunterteil mit einer Wasserkammer zum Befeuchten und/oder Erwärmen eines Atemgases, wobei der Atemgasbefeuchter einen Atemgaseinlass und einen Atemgasauslass umfasst, wobei zwischen dem Atemgaseinlass und dem Atemgasauslass ein Atemgaspfad zum Leiten des Atemgases vom Atemgaseinlass zum Atemgasauslass ausgebildet ist, der durch die Wasserkammer geführt ist, wobei der Atemgaspfad einen wasserdichten Kanal zum Leiten des befeuchteten und/oder angewärmten Atemgases umfasst, der an einem Ende in die Wasserkammer mündet und an einem anderen Ende in den Atemgasauslass mündet, wobei der Kanal derart ausgebildet ist, dass der Atemgasauslass am Gehäuseunterteil angeordnet und/oder zumindest teilweise durch dieses ausgebildet ist.

Unter "Atemgas" kann ein reines Atemgas oder ein Atemgasgemisch aus mehreren Atemgasen verstanden werden, mit dem ein Patient beatmet werden kann. Das Atemgas kann normale Umgebungsluft, Sauerstoff, Stickstoff, Kohlendioxid, Anästhesiegas, Wasserdampf, oder eine Kombination aus mindestens zwei dieser Komponenten sein oder umfassen.

Unter "Atemgasbefeuchtung" kann verstanden werden, dass das Atemgas durch eine kontrollierte Zugabe von Wasser bzw. einer wässrigen Lösung, vorzugsweise in Kombination mit einer Erwärmung des Atemgases, befeuchtet wird. Dies kann dadurch realisiert sein, dass das Atemgas derart über eine Flüssigkeitsoberfläche geleitet, dass die Flüssigkeit Wasser oder Wasserdampf an das Atemgas abgibt.

Der Begriff "Wasserkammer" soll hierin allgemein ein Behältnis zum Speichern einer Flüssigkeit (Wasser oder wasserhaltige Flüssigkeit) bezeichnen. Die Wasserkammer kann ein elektrisch steuerbares Heizelement, beispielsweise einen Heizstab oder eine Heizplatte, zum Erwärmen der Flüssigkeit umfassen, so dass die Flüssigkeit gezielt verdunstet und von dem Atemgas aufgenommen werden kann. In der Wasserkammer kann ferner ein Strömungsleitelement angeordnet sein, das ausgebildet ist, um das Atemgas in Richtung der Flüssigkeitsoberfläche zu leiten. Die Wasserkammer kann Füllstandanzeigen umfassen, die eine maximale und/oder eine minimale Füllhöhe für die Flüssigkeit anzeigen. Die Wasserkammer kann einen Wasserkammereinlass und einen Wasserkammerauslass aufweisen. Das Atemgas kann durch den Wasserkammereinlass in die Wasserkammer hinein und durch den Wasserkammerauslass aus der Wasserkammer herausgeführt werden.

Als "Atemgaspfad" sollen diejenigen Bereiche des Atemgasbefeuchter verstanden werden, die eingerichtet sind, das Atemgas aufzunehmen und/oder zu leiten. Der Atemgaspfad kann, zumindest größtenteils, innerhalb des Gehäuses des Atemgasbefeuchters ausgebildet sein. Der Atemgaspfad kann beispielsweise durch Kanäle, Kammern, (Durch-)Gänge, Öffnungen und dergleichen ausgebildet sein, die pneumatisch miteinander in Verbindung stehen. Der Atemgaspfad ist - abgesehen vom Einlass und vom Auslass - atemgasdicht zu seiner Umgebung ausgebildet.

Der Atemgaseinlass kann der Beginn des Atemgaspfades sein oder umfassen. Der Atemgaseinlass kann ausgebildet sein, um das Atemgas in den Atemgasbefeuchter einzuleiten. Der Atemgasauslass kann das Ende des Atemgaspfades sein oder umfassen. Der Atemgasauslass kann ausgebildet sein, um das Atemgas aus dem Atemgasbefeuchter auszuleiten.

Über den Atemgaseinlass und/oder den Atemgasauslass kann der Atemgasbefeuchter an weitere Kanäle, Schläuche und/oder an Komponenten eines Beatmungsgerätes luftdicht angeschlossen werden. Atemgaseinlass und Atemgasauslass können somit als Anschlüsse dienen, über die der Atemgasbefeuchter mit weiteren Komponenten luftdicht verbunden werden kann.

Von dem Atemgasauslass aus kann das erwärmte und/oder befeuchtete Atemgas direkt zum Patienten geführt werden. Der Atemgasauslass kann auch an ein Beatmungsgerät angeschlossen werden, so dass das erwärmte und/oder befeuchtete Atemgas über das Beatmungsgerät zum Patienten geführt werden kann. Die Verbindung mit dem Patienten kann über einen oder mehrere Beatmungsschläuche und/oder ein geeignetes Patienteninterface realisiert sein, die an den Atemgasauslass und/oder an das Beatmungsgerät angeschlossen werden können.

Unter "Patienteninterface" kann im Sinne der Erfindung jegliches Peripheriegerät verstanden werden, welches zur Interaktion mit einem Lebewesen ausgebildet ist. Das Patienteninterface kann beispielsweise eine Atemmaske, wie eine Nasenmaske, Nasenpolstermaske, Nasenbrille bzw. Sauerstoffbrille, Full-Face- oder Totalface-Maske, oder auch ein Trachealtubus oder eine Trachealkanüle sein.

Unter einem Beatmungsgerät können sämtliche Geräte verstanden werden, die ein Lebewesen bei der natürlichen Atmung unterstützen und/oder die Beatmung übernehmen und/oder einer Atemtherapie dienen und/oder einer Inhalationsnarkose dienen und/oder anderweitig auf die Atmung des Patienten einwirken. Ein Beatmungsgerät kann zum Beispiel ein Beatmungsgerät für klinische oder Heimanwendungen, ein Atemtherapiegerät, ein CPAP-, APAP- oder BiLevel-Gerät, ein Highflow-Therapiegerät, ein Narkose- bzw. Anästhesiegerät, ein Notfall-Beatmungsgerät, ein Sauerstofflieferndes Gerät, ein Diagnosesystem oder ein Hustentherapiegerät bzw. eine Hustenmaschine sein.

Dadurch, dass der Atemgasauslass am Gehäuseunterteil des Atemgasbefeuchters angeordnet und/oder durch dieses ausgebildet ist, können auch an den Atemgasauslass des Atemgasbefeuchters angeschlossene Komponenten am Gehäuseunterteil ansetzen. Dies bietet den Vorteil, dass die an den Atemgasauslass angeschlossenen Komponenten den Atemgasbefeuchter bei einer Zugbewegung nicht oder nur sehr erschwert aus dem Stand bringen können. Die Gefahr des Kippens oder Umfallens des Atemgasbefeuchters bei einem Zug am unteren Teil des Gerätes ist minimiert. Dies bietet somit allgemein den Vorteil einer guten Standfestigkeit des Atemgasbefeuchters.

Gemäß einer Ausführungsform kann der Atemgasauslass - wenn die Wasserkammer bestimmungsgemäß mit einer Flüssigkeit zum Befeuchten des Atemgases befüllt ist - unterhalb der Flüssigkeitsoberfläche angeordnet sein.

Gemäß einer Ausführungsform kann der Kanal zumindest abschnittweise durch das Gehäuseoberteil und/oder das Gehäuseunterteil ausgebildet sein oder durch diese verlaufen.

Unter "Kanal" kann ein längliches Rohr, ein Schlauch oder eine Kombination aus beidem verstanden werden. Der Kanal kann eine starre oder flexible oder teilweise flexible Kanalwand aufweisen und zur Leitung des Atemgases ausgebildet sein. Der Kanal ist als länglicher Hohlraum mit einer ein Lumen des Kanals umschließenden Wandung ausgebildet. Diese Wandung kann von dem Gehäuseoberteil und/oder dem Gehäuseunterteil ausgebildet sein.

Das Gehäuseoberteil und das Gehäuseunterteil können insbesondere zweiteilig ausgebildet sein. Um den Atemgasbefeuchter in einen Betriebszustand zu bringen, können das Gehäuseoberteil und das Gehäuseunterteil atemgasdicht miteinander verbunden werden. Die Verbindung kann bevorzugt reversibel ausgestaltet sein, so dass der Atemgasbefeuchter für eine Reinigung und/oder eine Befüllung auseinandergenommen werden kann. Die Verbindung kann über einen reversibel ausgestalteten Verbindungsmechanismus realisiert sein. Der Atemgasbefeuchter kann ferner eine Verriegelungsvorrichtung aufweisen, mit der die Verbindung der einzelnen Gehäuseteile atemgasdicht verriegelt werden kann.

Gemäß einer Ausführungsform kann der Atemgasbefeuchter ein Mittelteil aufweisen, das atemgasdicht zwischen dem Gehäuseoberteil und dem Gehäuseunterteil angeordnet ist, wobei der Kanal zumindest abschnittweise durch das Mittelteil ausgebildet sein oder durch dieses verlaufen kann. Gemäß einer Ausführungsform kann der Kanal zumindest abschnittsweise erst durch die Verbindung des Gehäuseoberteils mit dem Gehäuseunterteil oder durch die Verbindung des Gehäuseoberteils mit dem Gehäuseunterteil und dem Mittelteil ausgebildet sein.

Der Kanal bzw. die Kanalwand kann zumindest abschnittsweise einteilig oder mehrteilig, beispielsweise zweiteilig, ausgebildet sein. Dies kann zum einen die Herstellung und zum anderen eine Reinigung des Kanals erleichtern. Bei einer Aufhebung der Verbindung von Gehäuseoberteil, Gehäuseunterteil und/oder Mittelteil kann der Kanal somit zumindest bereichsweise geöffnet werden, insbesondere um diesen zu reinigen.

Gemäß einer Ausführungsform kann der Kanal ferner eine Heizeinrichtung umfassen, die ausgebildet sein kann, um den Kanal auf eine erste Temperatur zum Vermeiden von Kondensation und/oder auf eine zweite Temperatur zum Abtöten und/oder Inaktivieren von Keimen zu erwärmen. Der Kanal kann somit beispielsweise einen Heizdraht umfassen, der entlang des Kanals verläuft und Wärme in das Innere des Kanals abgeben kann.

Gemäß einer Ausführungsform kann der Kanal in seiner Längsrichtung betrachtet gerade und/oder zumindest teilweise gewinkelt und/oder gebogen ausgebildet sein. Gemäß einer Ausführungsform kann der Kanal in seiner Längsrichtung betrachtet einen oder mehrere fluidisch miteinander in Verbindung stehende Kanalabschnitte umfassen, wobei der Kanal einen ersten Kanalabschnitt zur Verbindung mit dem Wasserkammerauslass, einen zweiten Kanalabschnitt zur Verbindung mit dem Atemgasauslass und mindestens einen den ersten Kanalabschnitt mit dem zweiten Kanalabschnitt verbindenden weiteren Kanalabschnitt umfassen kann, wobei die Kanalabschnitte ausgebildet sein können, um mindestens eine Umlenkung des Atemgases zu bewirken.

Eine Umlenkung des Atemgases kann eine Schallentwicklung unterdrücken. Die Anzahl an Kanalabschnitten kann variabel gewählt sein. Dabei gilt, dass je mehr Umlenkungen das Atemgas erfährt, desto schallärmer kann der Atemgasbefeuchter ausgebildet sein.

Gemäß einer Ausführungsform kann der Kanal so ausgebildet sein, dass - wenn der Atemgasbefeuchter bestimmungsgemäß auf einem Boden des Gehäuseunterteils steht - der erste Kanalabschnitt zumindest größtenteils vertikal angeordnet ist und/oder der zweite Kanalabschnitt zumindest größtenteils waagerecht angeordnet ist und/oder ein dritter Kanalabschnitt zumindest größtenteils waagerecht angeordnet ist und/oder ein vierter Kanalabschnitt zumindest größtenteils vertikal angeordnet ist. Gemäß einer Ausführungsform kann der erste Kanalabschnitt so ausgebildet sein, dass er - wenn der Atemgasbefeuchter bestimmungsgemäß auf dem Boden steht und die Wasserkammer mit einer Flüssigkeit zum Befeuchten des Atemgases befüllt ist - in vertikaler Richtung von der Flüssigkeitsoberfläche wegführt.

Der Kanal ist vorzugsweise so ausgebildet, dass keine überschüssige Flüssigkeit von dem Atemgasstrom mitgerissen werden kann. Der Kanal kann zudem ausgebildet sein, dass die Flüssigkeit bei einem Verkippen des Atemgasbefeuchters nicht - oder nur sehr erschwert - aus der Wasserkammer in den Kanal fließen kann. Zu diesem Zwecke kann der erste Kanalabschnitt vertikal angeordnet sein. Der erste Kanalabschnitt kann derart ausgebildet sein, dass dieser - wenn der Atemgasbefeuchter bestimmungsgemäß auf einem Boden des Gehäuseunterteils steht - orthogonal zur Flüssigkeitsoberfläche angeordnet ist.

Gemäß einer Ausführungsform kann der Atemgasbefeuchter so ausgebildet sein, dass der Atemgaseinlass - wenn der Atemgasbefeuchter bestimmungsgemäß auf einem Boden des Gehäuseunterteils steht - in vertikaler Richtung betrachtet oberhalb des Atemgasauslass angeordnet ist. Gemäß einer Ausführungsform kann der Atemgasbefeuchter so ausgebildet sein, dass der dritte Kanalabschnitt - wenn der Atemgasbefeuchter bestimmungsgemäß auf einem Boden des Gehäuseunterteils steht -in vertikaler Richtung betrachtet oberhalb des Atemgasauslasses und/oder des zweiten Kanalabschnitts angeordnet ist. Eine derartige Anordnung bietet einen zusätzlichen Schutz vor einem unkontrollierten Eindringen von Wasser in den Kanal und/oder in den Atemgasauslass.

Gemäß einer Ausführungsform kann der Kanal in seiner Querrichtung betrachtet gerade und/oder zumindest teilweise gewinkelt und/oder gebogen ausgebildet sein. Somit kann der Atemgaspfad aus der Mitte des Atemgasbefeuchters hin zum Gehäuse und/oder zum Atemgasauslass führen. Durch eine Abwinkelung oder ein Abbiegen des Kanals in Querrichtung kann der Atemgasbefeuchter besonders platzsparend ausgebildet sein. Der Kanal kann eingerichtet sein, um das Atemgas beim Betrieb des Atemgasbefeuchters von dem Wasserkammerauslass zum Atemgasauslass zu befördern. Eine Biegung des Kanals in seiner Querrichtung betrachtet kann besonders vorteilhaft sein, da Turbulenzen in der Luftführung vermieden werden können und ein (möglichst) laminarer Luftstrom im Kanal entstehen kann.

Gemäß einer Ausführungsform kann der Kanal zumindest abschnittweise ein Trennelement aufweisen, das ausgebildet ist, um den Kanal in seiner Längsrichtung betrachtet zumindest abschnittweise in mindestens zwei Teilkanäle zu unterteilen. Das Trennelement kann ausgebildet sein, um die Strömung des Atemgases im Kanal zu beruhigen. Das Trennelement kann ausgebildet sein, um eine laminare Strömung im Kanal zu begünstigen.

Gemäß einer Ausführungsform kann der Atemgasbefeuchter einen Wasserkammereinlass umfassen, der im Gehäuseoberteil oder im Mittelteil ausgebildet sein kann, wobei der Wasserkammereinlass als einfache Öffnung oder als Wasserkammereinlasskanal ausgebildet sein kann. Der Wasserkammereinlass kann in manchen Ausführungsformen als Öffnung im Gehäuseoberteil ausgebildet sein. Gemäß einer Ausführungsform kann der Wasserkammereinlass dem Atemgaseinlass entsprechen. In alternativen Ausführungsformen kann der Wasserkammereinlass auch als Öffnung im Mittelteil ausgebildet sein. Sodann kann der Atemgaseinlass im Gehäuseoberteil angeordnet oder durch dieses ausgebildet sein. In einer solchen Ausführungsform kann das Atemgas zunächst über den Atemgaseinlass in das Gehäuseoberteil eingeleitet werden und von dort über den Wasserkammereinlass des Mittelteils in die Wasserkammer. In manchen Ausführungsformen kann der Wasserkammereinlass ein Volumen aufweisen und als Wasserkammereinlasskanal im Gehäuseoberteil und/oder im Mittelteil ausgebildet sein.

Gemäß einer Ausführungsform kann der Atemgasbefeuchter eine Kammer umfassen, die zwischen dem Atemgaseinlass und dem Wasserkammereinlass ausgebildet und Teil des Atemgaspfades sein kann, wobei ein Querschnitt der Kammer größer als ein Querschnitt des Atemgaseinlasses und/oder ein Querschnitt des Wasserkammereinlasses oder des Wasserkammereinlasskanals und/oder des Kanals sein kann. Gemäß einer Ausführungsform kann die Kammer und/oder der Kanal und/oder die Wasserkammer und/oder der Wasserkammereinlass oder Wasserkammereinlasskanal akustische Impedanzen aufweisen und derart unterschiedlich dimensioniert sein, so dass ihre jeweiligen akustischen Impedanzen voneinander abweichen.

Die genannten unterschiedlichen Abschnitte des Atemgaspfades können sich jeweils von unmittelbar angrenzenden Abschnitten des Atemgaspfades, insbesondere hinsichtlich einer Größe ihrer Querschnitte orthogonal zur Durchflussrichtung, unterscheiden. Dabei ist es bevorzugt, wenn sich ein Querschnitt und/oder eine Form des Atemgaspfades an einem Übergang zwischen zwei angrenzenden Abschnitten des Atemgaspfades, abrupt bzw. sprunghaft verkleinert oder vergrößert.

Durch die so entstehenden unterschiedlichen akustischen Impedanzen kann ein Schall gedämpft werden. Eine "akustische Impedanz", insbesondere eine akustische Flussimpedanz, benennt den Widerstand, der einer Schallausbreitung entgegengesetzt wird. Bei einem Übergang zwischen Bereichen mit unterschiedlicher akustischer Impedanz kommt es zu Reflexionen der Schallwellen, wodurch eine schalldämpfende Wirkung erreicht wird.

Gemäß einer Ausführungsform kann der Wasserkammerauslass und/oder der erste Kanalabschnitt in einer horizontalen Ebene betrachtet im Bereich der Mitte des Atemgasbefeuchters angeordnet sein. Der Atemgasbefeuchter ist so eingerichtet, dass der Wasserkammerauslass und/oder der erste Kanalabschnitt beabstandet von der Flüssigkeitsoberfläche der Wasserkammer angeordnet ist. Die maximale Füllhöhe ist derart gewählt, dass die Flüssigkeit nicht den Wasserkammerauslass erreicht. Zudem ist der Wasserkammerauslass und/oder der erste Kanalabschnitt mittig im Gerät angeordnet, so dass die Flüssigkeit während des Betriebes und/oder bei Transport nicht unkontrolliert in den Kanal eindringen kann.

Der Atemgasbefeuchter ist als dreidimensionaler Körper ausgebildet und kann einen Boden und mindestens eine Seitenwand umfassen. Der Atemgasbefeuchter kann beispielsweise die Grundform eines Quaders, Würfels, Prismas, Zylinders, einer Pyramide oder dergleichen aufweisen. Der Wasserkammerauslass kann derart angeordnet sein, dass er in horizontaler Ebene maximal weit entfernt zur Seitenwand bzw. zu allen Seitenwänden des Atemgasbefeuchters ist. Der Wasserkammerauslass kann in horizontaler Ebene betrachtet im Mittelpunkt des Atemgasbefeuchters angeordnet sein. Der Atemgasbefeuchter kann somit derart eingerichtet sein, dass er bis zu einem gewissen Grad geneigt werden kann, ohne dass Flüssigkeit in den Wasserkammerauslass und/oder den Kanal gelangen kann. Durch die Anordnung des Wasserkammerauslasses in der Mitte des Atemgasbefeuchters kann dieser derart eingerichtet sein, dass eine Neigung oder ein Kippen des Befeuchters nach allen Seiten tolerierbar ist, ohne dass Flüssigkeit in den oberen Bereich des Atemgasbefeuchters gelangen kann.

Die Erfindung betrifft zudem ein Beatmungsgerät mit einem Atemgasweg, einem ersten Anschluss und einem zweiten Anschluss, umfassend einen Atemgasbefeuchter nach einem der vorhergehenden Ansprüche, wobei der Atemgasbefeuchter über einen Atemgaseinlass an den ersten Anschluss und über einen Atemgasauslass an den zweiten Anschluss an den Atemgasweg das Beatmungsgerät angeschlossen ist.

Unter "Beatmungsgerät" kann eine Vorrichtung zur Beatmung und/oder zur Narkotisierung verstanden werden. Das Beatmungsgerät kann somit eine Beatmungsfunktion und eine Anästhesiefunktion und eine Kombination aus diesen aufweisen. Unter "Beatmung" kann eine künstliche Beatmung, eine Atemunterstützung, eine Atemtherapie, eine Atemdiagnose, eine Hustenunterstützung, eine Sauerstoffliefernde Therapie wie beispielweise eine High-Flow-Therapie, eine Inhalationsnarkose und eine Kombination aus mindestens zwei dieser Beispiele verstanden werden. Somit kann ein Individuum unter Verwendung des Beatmungsgerätes beatmet oder in der Atmung unterstützt werden und alternativ oder zusätzlich auch unter Narkose gehalten werden. Zu diesem Zwecke kann das Beatmungsgerät auch mit Anästhesiegasen betrieben werden und somit einer Inhalationsanästhesie dienen.

Unter einem Beatmungsgerät können somit sämtliche Geräte verstanden werden, die ein Individuum bei der natürlichen Atmung unterstützen und/oder die Beatmung übernehmen und/oder einer Atemtherapie dienen und/oder einer Inhalationsnarkose dienen und/oder anderweitig auf die Atmung des Patienten oder Anwenders einwirken. Ein Beatmungsgerät kann für klinische oder Heimanwendungen ausgebildet sein und beispielsweise ein Atemtherapiegerät, ein CPAP-, APAP- oder BiLevel-Gerät, ein Highflow-Therapiegerät, ein Narkose- bzw. Anästhesiegerät, ein Notfall-Beatmungsgerät, ein Sauerstofflieferndes Gerät, ein Diagnosesystem oder ein Hustentherapiegerät bzw. eine Hustenmaschine sein.

Unter "Patienteninterface" kann im Sinne der Erfindung jegliches Peripheriegerät verstanden werden, welches zur Interaktion mit einem Lebewesen ausgebildet ist. Insbesondere ist das Patienteninterface zu Beatmungs-, Therapie- und/oder Diagnosezwecken in Verbindung mit dem Beatmungsgerät ausgebildet. Das Patienteninterface kann als Atemmaske ausgebildet sein. Darunter fallen zum Beispiel, aber nicht ausschließlich, Nasenmasken, Nasenpolstermasken, Nasenbrillen bzw. Sauerstoffbrillen, Full-Face- oder Totalface-Masken sowie Trachealtuben bzw. - kanülen.

### Kurze Beschreibung der Figuren

Im Folgenden werden Ausführungsformen der Erfindung mit Bezug auf die beigefügten Figuren beschrieben. Weder die Beschreibung noch die Figuren sind als Beschränkung des Umfangs der Erfindung zu verstehen.
Fig. 1 zeigt eine schematische Abbildung eines Atemgasbefeuchters im Querschnitt gemäß einer Ausführungsform der Erfindung.
Fig. 2 zeigt eine perspektivische Außenansicht eines Atemgasbefeuchters gemäß einer Ausführungsform der Erfindung.
Fig. 3 zeigt einen perspektivischen Querschnitt eines Atemgasbefeuchters gemäß einer Ausführungsform der Erfindung.
Fig. 4 zeigt eine perspektivische Außenansicht eines Atemgasbefeuchters ohne Darstellung des Gehäuseoberteils gemäß einer Ausführungsform der Erfindung.
Fig. 5 zeigt eine perspektivische Außenansicht eines Mittelteils eines Atemgasbefeuchters gemäß einer Ausführungsform der Erfindung.
Fig. 6 zeigt eine Ansicht eines Mittelteils von oben gemäß einer Ausführungsform der Erfindung.
Fig. 7 zeigt eine Ansicht eines Gehäuseunterteils von oben gemäß einer Ausführungsform der Erfindung.

Die Figuren sind rein schematisch und mitunter nicht maßstabsgetreu. Werden in verschiedenen Zeichnungen gleiche Bezugszeichen verwendet, so bezeichnen diese Bezugszeichen gleiche oder gleichwirkende Merkmale.

### Ausführungsformen der Erfindung

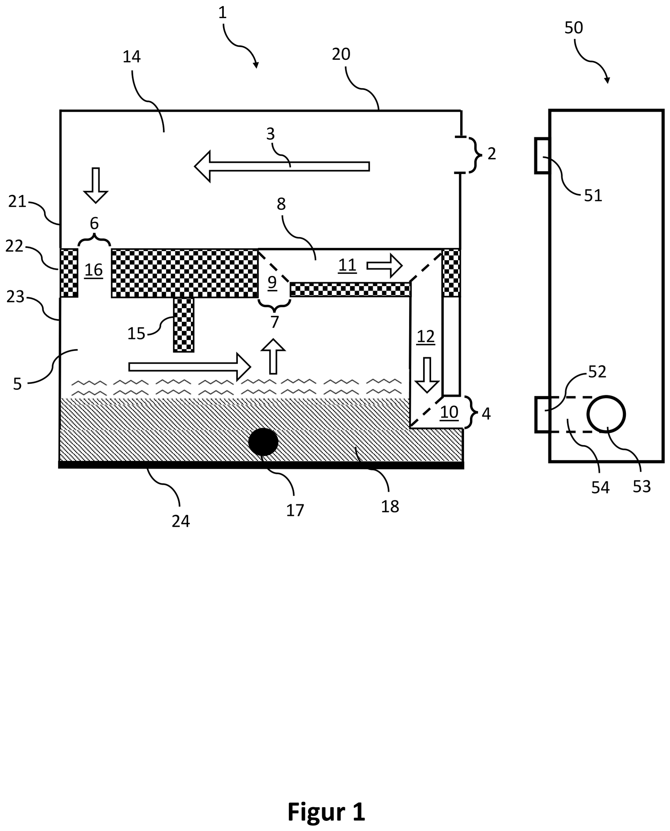

Fig. 1 zeigt eine schematische Abbildung eines Atemgasbefeuchters 1 im Querschnitt gemäß einer Ausführungsform der Erfindung. Der Atemgasbefeuchter 1 ist zur Verwendung in Kombination mit einem Beatmungsgerät 50 ausgebildet. Der Atemgasbefeuchter 1 ist zum Befeuchten und/oder Erwärmen eines Atemgases ausgebildet. In diesem Beispiel weist der Atemgasbefeuchter 1 ein zweigeteiltes Gehäuse 20 bestehend aus einem Gehäuseunterteil 23 und einem Gehäuseoberteil 21 auf. Das Gehäuseunterteil 23 umfasst einen Boden 24, der ausgebildet ist, um den Atemgasbefeuchter 24 standsicher aufzustellen. Der Boden 24 kann wie skizziert plan ausgebildet sein. Der Boden 24 kann beispielsweise auch in Form von einem oder mehreren Füßen ausgebildet sein, mit denen der Atemgasbefeuchter 1 aufgestellt werden kann. Der Boden 24 definiert eine Unterseite des Atemgasbefeuchters 1. In einem bestimmungsgemäßen Gebrauch steht der Atemgasbefeuchter 1 auf dem Boden 24 des Gehäuseunterteils 23.

Ferner umfasst der Atemgasbefeuchter 1 einen Atemgaseinlass 2 und einen Atemgasauslass 4. Der Atemgaseinlass 2 kann in einem bestimmungsgemäßen Gebrauch in vertikaler Richtung betrachtet oberhalb des Atemgasauslass 4 angeordnet sein. Zwischen dem Atemgaseinlass 2 und dem Atemgasauslass 5 ist ein Atemgaspfad 3 zum Leiten des Atemgases ausgebildet. Der Atemgaspfad 3 ist in Fig. 1 mit Hilfe von Blockpfeilen angedeutet. Die Pfeile verdeutlichen die Strömungsrichtung des Atemgases. Der Atemgasbefeuchter 1 ist ausgebildet, um das Atemgas entlang des Atemgaspfades 3 zu führen.

Der Atemgaseinlass 2 kann zum Anschließen an eine Vorrichtung mit einem Atemgasantrieb ausgebildet sein. Eine solche Vorrichtung wird im Folgenden als Beatmungsgerät 50 bezeichnet. Das Beatmungsgerät 50 kann einen ersten Anschluss 51 zum Anschließen an den Atemgaseinlass 2 umfassen. Somit kann das Atemgas aus dem Beatmungsgerät 50 über den Atemgaseinlass 2 ins Innere des Atemgasbefeuchters 1 strömen. Das Atemgas kann somit in den Atemgaspfad 3 eintreten. Über den Atemgasauslass 4 kann das befeuchtete und/oder erwärmte Atemgas aus dem Inneren des Atemgasbefeuchters 1 ausströmen. Das Atemgas kann somit aus dem Atemgaspfad 3 austreten und für die Beatmung eines Patienten verwendet werden.

Der Atemgasauslass 4 kann ebenfalls zum Anschließen an das Beatmungsgerät 50 ausgebildet sein. Sodann kann das Atemgas aus dem Inneren des Atemgasbefeuchters 1 zurück in das Beatmungsgerät 50 strömen und von dort zur Beatmung des Patienten verwendet werden. Zu diesem Zweck kann das Beatmungsgerät 50 einen zweiten Anschluss 52 zum Anschließen an den Atemgasauslass 4 umfassen. Zudem kann das Beatmungsgerät 50 einen Schlauchanschluss 53 und eine Leitung 54 aufweisen, die zwischen dem zweiten Anschluss 52 und dem Schlauchanschluss 53 ausgebildet ist. Bevor das Atemgas zum Patienten geleitet wird, kann üblicherweise der Druck und/oder der Atemgasfluss (Flow) und/oder das Volumen gemessen werden. Zu diesem Zweck kann der Atemgasbefeuchter 1 und/oder das Beatmungsgerät 50 entsprechende Sensoren aufweisen.

Das Atemgas kann in alternativen Ausführungsformen auch direkt aus dem Atemgasauslass 4 zur Beatmung des Patienten verwendet werden und zu diesem Zwecke als Schlauchanschluss ausgebildet sein.

Die Verbindung mit dem Patienten kann über einen oder mehrere hier nicht dargestellte Beatmungsschläuche und/oder ein geeignetes Patienteninterface realisiert sein. Die Beatmungsschläuche und/oder das Patienteninterface können üblicherweise an den Schlauchanschluss 53 des Beatmungsgerätes 50 angeschlossen werden. In manchen Ausführungsformen können die Beatmungsschläuche und/oder das Patienteninterface auch direkt an den Atemgasauslass 4 des Atemgasbefeuchters 1 angeschlossen werden. Das Patienteninterface kann beispielsweise eine Atemmaske, ein Trachealtubus oder eine Trachealkanüle sein, mit der das erwärmte und/oder befeuchtet Atemgas aus dem Atemgasbefeuchter 1 dem Atemapparat des Patienten zugeführt werden kann.

Der Atemgasauslass 4 kann am Gehäuseunterteil 23 angeordnet und/oder durch dieses ausgebildet sein. Der Atemgasauslass 4 kann - wenn der Atemgasbefeuchter 1 bestimmungsgemäß auf dem Boden 24 des Gehäuseunterteils 23 steht - unterhalb der Flüssigkeitsoberfläche angeordnet sein. Der Atemgasauslass 4 ist vorzugsweise benachbart zum Boden 24 angeordnet. Der Atemgasauslass 4 ist in vertikaler Richtung betrachtet derart tief angeordnet, dass eine Zugkraft, die an dieser ansetzt, das Gerät nicht oder nur erschwert zum Wackeln oder Kippen bringen kann. Der Atemgasauslass 4 ist in vertikaler Richtung betrachtet derart tief angeordnet, dass auch der Schlauchanschluss 53 des Beatmungsgerätes tief angeordnet sein kann. Somit kann die Leitung 54 im Gerät sehr kurz ausgebildet sein, und insbesondere ohne eine vertikale Umlenkung auskommen.

Um das Atemgas zu befeuchten, wird das Atemgas entlang des Atemgaspfades 2 durch eine Wasserkammer 5 geführt. Die Wasserkammer 5 ist ausgebildet, um dem Atemgas eine Feuchtigkeit und/oder eine Temperatur zuzuführen. Das Gehäuseunterteil 23 kann als Wasserkammer 5 ausgebildet sein oder die Wasserkammer 5 umfassen. Die Wasserkammer 5 ist ein Behältnis zum Speichern einer Flüssigkeit 18, mit der das Atemgas befeuchtet werden kann. Die Flüssigkeit 18 kann beispielsweise Wasser oder eine wasserhaltige Flüssigkeit sein. Die Wasserkammer 5 kann ein elektrisches Heizelement 17 zum Erwärmen der Flüssigkeit 18 aus der Wasserkammer 5 aufweisen. Der Atemgasbefeuchter 1 und/oder das Beatmungsgerät 50 kann eine hier nicht dargestellte Steuereinrichtung umfassen, mit der das elektrische Heizelement 17 zur Vorgabe einer bestimmten Temperatur angesteuert werden kann. Das Heizelement 17 kann beispielsweise als elektrischer Heizstab ausgebildet sein, der in die Flüssigkeit 18 eingeführt werden kann.

Die Wasserkammer 5 kann ausgebildet sein, um die Atemgasströmung möglichst dicht über die Flüssigkeitsoberfläche zu leiten. Zu diesem Zweck kann in der Wasserkammer mindestens ein Strömungsleitelement 15 angeordnet sein, das ausgebildet ist, um das Atemgas in Richtung der Flüssigkeitsoberfläche zu leiten. Der Atemgasbefeuchter 1 ist so ausgebildet, dass das Atemgas entlang des Atemgaspfades 3 vom Atemgaseinlass 2 zum Atemgasauslass 4 strömt. Dabei kommt das Atemgas in Kontakt mit der Flüssigkeit 18 im verdampften und erwärmten Zustand, wodurch das Atemgas befeuchtet und/oder temperiert wird.

Um eine Atemgasströmung im Atemgasbefeuchter 1 zu erzeugen, kann der Atemgasbefeuchter 1 mindestens einen hier nicht dargestellten Atemgasantrieb, beispielsweise ein Gebläse umfassen. Der Atemgasantrieb kann im oder am Gehäuse des Atemgasbefeuchters 1 angeordnet sein. Somit kann der Atemgasbefeuchter 1 selbst als Beatmungsgerät 50 ausgebildet sein. Es ist jedoch bevorzugt, dass die Atemgasströmung alternativ oder zusätzlich von einem Atemgasantrieb des Beatmungsgerätes 50 erzeugt wird.

Um den Betrieb des Atemgasbefeuchters 1, insbesondere das Heizelement 17, zu steuern kann der Atemgasbefeuchter 1 eine hier nicht dargestellte Steuereinrichtung umfassen. Die Steuereinrichtung kann im oder am Gehäuse des Atemgasbefeuchters 1 angeordnet sein. Es ist jedoch auch möglich, dass der Atemgasbefeuchter 1 alternativ oder zusätzlich von einer Steuereinrichtung des Beatmungsgerätes 50 gesteuert wird.

Die Wasserkammer 5 kann einen Wasserkammereinlass 6 und einen Wasserkammerauslass 7 aufweisen. Durch diese kann das Atemgas in die Wasserkammer 5 ein- und wieder ausströmen. Zwischen dem Wasserkammerauslass 7 und dem Atemgasauslass 4 ist ein Kanal 8 zum Leiten des befeuchteten und/oder angewärmten Atemgases zum Atemgasauslass 4 ausgebildet. Der Kanal 8 kann zumindest abschnittweise durch das Gehäuseoberteil 21 und/oder das Gehäuseunterteil 23 ausgebildet sein oder durch diese verlaufen.

Der Atemgasbefeuchter 1 kann - wie in diesem Beispiel gezeigt - ferner ein Mittelteil 22 aufweisen (In Fig. 1 kariert dargestellt). Das Mittelteil 22 kann atemgasdicht zwischen dem Gehäuseoberteil 21 und dem Gehäuseunterteil 23 angeordnet sein. Der Kanal 8 kann zumindest abschnittweise durch das Mittelteil 22 ausgebildet sein oder durch dieses verlaufen. Der Kanal 8 kann zumindest abschnittsweise (erst) durch eine Verbindung des Gehäuseoberteils 21 mit dem Gehäuseunterteil 23 oder durch eine Verbindung des Gehäuseoberteils 21 mit dem Gehäuseunterteil 23 und dem Mittelteil 22 ausgebildet sein.

Gehäuseoberteil 21, Gehäuseunterteil 23 und/oder Mittelteil 22 können reversibel miteinander verbunden werden. Die Gehäuseteile 21,22,23 können somit beispielsweise für eine Reinigung voneinander getrennt und anschließend wieder miteinander verbunden werden. Durch die Trennung der Gehäuseteile 21,22,23 kann vorzugsweise auch der Kanal 8 zumindest bereichsweise geöffnet werden, um diesen einfacher reinigen zu können. Der Kanal 8 kann in manchen Ausführungsformen eine (zusätzliche) Heizeinrichtung umfassen, die ausgebildet ist, um den Kanal 8 auf eine erste Temperatur zum Vermeiden von Kondensation und/oder auf eine zweite Temperatur zum Abtöten und/oder Inaktivieren von Keimen zu erwärmen (nicht gezeigt).

Der Kanal 8 kann in seiner Längsrichtung betrachtet zumindest teilweise gerade und/oder zumindest teilweise gewinkelt und/oder gebogen ausgebildet sein. Wie in diesem Beispiel gezeigt, kann der Kanal 8 in seiner Längsrichtung betrachtet einen oder mehrere fluidisch miteinander in Verbindung stehende Kanalabschnitte 9, 10, 11, 12 umfassen.

Ein erster Kanalabschnitt 9 kann zur Verbindung mit dem Wasserkammerauslass 7 ausgebildet sein. Ein zweiter Kanalabschnitt 10 kann zur Verbindung mit dem Atemgasauslass 4 ausgebildet sein. Ferner können ein oder mehrere weitere Kanalabschnitte 11,12 zwischen dem ersten Kanalabschnitt 9 und dem zweiten Kanalabschnitt 10 angeordnet sein und diese miteinander verbinden. Die Kanalabschnitte 9,10,11,12 stehen pneumatisch miteinander in Verbindung und sind ausgebildet, um mindestens eine Umlenkung des Atemgases im Kanal 8 zu bewirken.

In diesem Beispiel kann der jeweilige Umlenkwinkel +/- 90° betragen, so dass sich vertikale und horizontale Kanalabschnitte abwechseln. Dabei kann der erste Kanalabschnitt 9 zumindest größtenteils vertikal angeordnet sein. Dadurch kann das befeuchtete und/oder erwärmte Atemgas zunächst in einer Vertikalen von der Flüssigkeitsoberfläche weggeleitet werden. Der sich anschließende dritte Kanalabschnitt 11 kann um 90° abgewinkelt zum ersten Kanalabschnitt 9 angeordnet sein, so dass dieser zumindest größtenteils waagerecht angeordnet ist.

Der sich an den dritten Kanalabschnitt 11 anschließende vierte Kanalabschnitt 12 kann wiederrum um 90° abgewinkelt zum vorherigen Kanalabschnitt 11 angeordnet sein, so dass dieser zumindest größtenteils vertikal angeordnet ist. Dadurch kann der Kanal 8 eingerichtet sein, das Atemgas vertikal in Richtung des Bodens 24 zu leiten. Der sich an den vierten Kanalabschnitt 12 anschließende zweite Kanalabschnitt 10 kann eine abermalige Umlenkung des Atemgases in die Waagerechte bewirken. Somit kann das Atemgas über den zweiten Kanalabschnitt 10 und den Atemgasauslass 4 aus dem Atemgasbefeuchter 1 entlassen werden.

In diesem Beispiel ist der zweite Kanalabschnitt 10, der zum Atemgasauslass 4 führt, in vertikaler Richtung betrachtet tiefer angeordnet als die übrigen Kanalabschnitte 9,11,12 und der Wasserkammerauslass 7. Der Kanal 8 ist somit ausgebildet, eine möglichst tiefe Anordnung des Atemgasauslasses 4 im Atemgasbefeuchter 1 zu realisieren. Der dritte Kanalabschnitt (11) kann in vertikaler Richtung betrachtet den am höchsten gelegenen Abschnitt des Kanals darstellen.

In einem einfachen Ausführungsbeispiel kann der Wasserkammereinlass 6 im Gehäuseoberteil 21 angeordnet sein. Sodann kann der Wasserkammereinlass 6 der Atemgaseinlass 2 sein (nicht gezeigt). Zwischen dem Wasserkammereinlass 6 und dem Atemgaseinlass 2 kann wie gezeigt auch eine Kammer 14 ausgebildet sein. Sodann kann der Atemgaspfad 3 von dem Atemgaseinlass 2 in die Kammer 14 und von dieser zum Wasserkammereinlass 6 und zur Wasserkammer 5 führen.

Der Wasserkammereinlass 6 kann als Öffnung zwischen dem Gehäuseoberteil 21 und dem Gehäuseunterteil 23 ausgebildet sein (nicht gezeigt). Der Wasserkammereinlass 6 kann wie in diesem Beispiel gezeigt auch als Öffnung im Mittelteil 22 bzw. zwischen dem Mittelteil 22 und dem Gehäuseunterteil 23 ausgebildet sein. Der Wasserkammereinlass 6 kann auch als Wasserkammereinlasskanal 16 mit einem eigenen Volumen ausgebildet sein.

In diesem Beispiel ist ein Querschnitt (orthogonal zur Durchflussrichtung) der Kammer 14 größer als ein Querschnitt des Atemgaseinlasses 2 und/oder ein Querschnitt des Wasserkammereinlasses 6 oder des Wasserkammereinlasskanals 16 und/oder ein Querschnitt des Kanals 8. Auch ein Querschnitt der Wasserkammer 5 ist in diesem Beispiel größer als der Querschnitt des Atemgaseinlasses 2 und/oder des Wasserkammereinlasses 6 oder des Wasserkammereinlasskanals 16 und/oder des Kanals 8. Somit unterscheiden sich die unmittelbar angrenzenden Abschnitte des Atemgaspfades 3 hinsichtlich der Größe ihres Querschnittes orthogonal zur Durchflussrichtung. Vorzugsweise vergrößert bzw. verkleinert sich der Querschnitt und/oder die Form des Atemgaspfades 3 an einem Übergang zwischen den angrenzenden Abschnitten abrupt oder sprunghaft. Die Kammer 14 und/oder der Kanal 8 und/oder die Wasserkammer 5 und/oder der Wasserkammereinlass 6 oder Wasserkammereinlasskanal 16 können derart unterschiedlich dimensioniert sein, so dass ihre jeweiligen akustischen Impedanzen voneinander abweichen. Dies kann zu einer Verringerung der Betriebsgeräusche des Atemgasbefeuchters 1 führen.

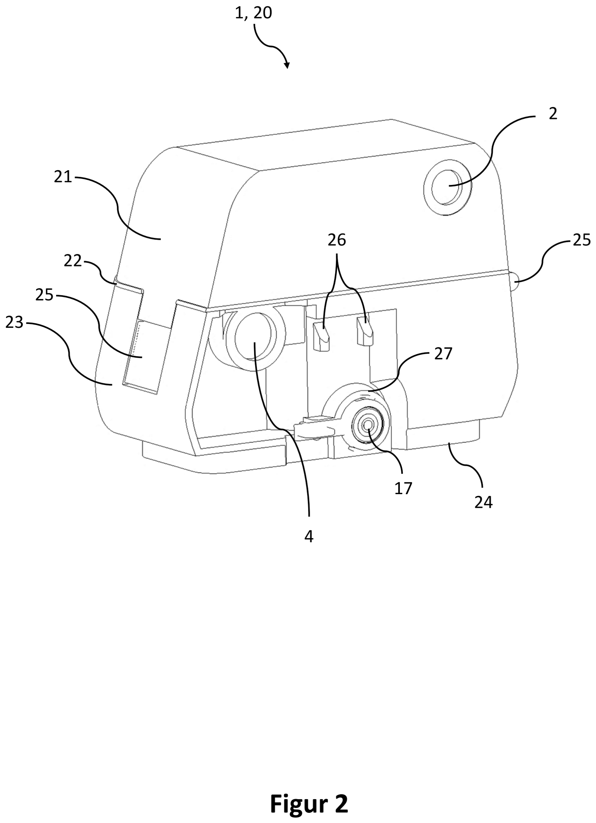

Fig. 2 zeigt eine perspektivische Außenansicht eines Atemgasbefeuchters gemäß einer Ausführungsform der Erfindung. Aus Fig. 2 wird ersichtlich, dass der Atemgasauslass 4 am Gehäuseunterteil 23 angeordnet ist. Der Atemgaseinlass 2 ist in diesem Beispiel am Gehäuseoberteil 21 angeordnet. Der Atemgasauslass 4 ist in diesem Beispiel in einer vertikalen Richtung betrachtet näher am Boden 24 angeordnet als der Atemgaseinlass 2. Das Heizelement 17 ist ebenfalls am Gehäuseunterteil 23 angeordnet. Das Gehäuseunterteil 23 kann eine Heizelement-Aufnahme 27 aufweisen, über die bzw. durch die das Heizelement 17 am Gehäuseunterteil 23 aufgenommen werden kann. Das Heizelement 17 kann über einen mechanischen Verschluss reversibel mit dem Gehäuseunterteil 23 verbunden sein. Beispielsweise kann das Heizelement 17 über einen Drehverschluss, vorzugsweise über einen Bajonettverschluss, in das Gehäuseunterteil eingefügt bzw. befestigt werden. In dem gezeigten Ausführungsbeispiel liegen der Atemgaseinlass 2 und der Atemgasauslass 4 an derselben Seitenfläche des Atemgasbefeuchters 1. Auch das Heizelement 17 kann an derselben Seitenfläche angeordnet sein.

Gehäuseoberteil 21, Gehäuseunterteil 23 und/oder Mittelteil 22 können reversibel miteinander verbunden werden. Die Verbindung ist vorzugsweise vollständig atemgasdicht. Zu diesem Zwecke kann der Atemgasbefeuchter 1 hier nicht dargestellte (elastomere) Dichtflächen aufweisen, die an den Kontaktflächen der unterschiedlichen Gehäuseteile 21,22,23 angeordnet sind. Gehäuseoberteil 21, Gehäuseunterteil 23 und/oder Mittelteil 22 können aus einem elastomeren Material gefertigt sein oder ein solches umfassen.

Zudem kann der Atemgasbefeuchter 1 über mindestens eine Verriegelungsvorrichtung 25 verfügen. Zur Montage kann das Gehäuseoberteil 21 auf das Gehäuseunterteil 23 aufgesetzt und optional verriegelt werden. Bei einer Ausführung mit Mittelteil 22 kann dieses zwischen Gehäuseoberteil 21 und Gehäuseunterteil 23 eingespannt werden. Zur Montage kann das Mittelteil 22 in das Gehäuseunterteil 23 eingesetzt werden. Sodann kann das Gehäuseoberteil 21 auf das Mittelteil 22 und/oder das Gehäuseunterteil 23 aufgesetzt und verriegelt werden.

An den Verbindungsstellen zwischen Gehäuseoberteil 21, Gehäuseunterteil 23 und/oder Mittelteil 22 können hier nicht dargestellte, elastomere Dichtflächen, beispielsweise aus Silikon, die Verbindung atemgasdicht gestalten.

Die Verriegelungsvorrichtung 25 kann Gehäuseoberteil 21 und Gehäuseunterteil 23 miteinander verriegeln. Die Verriegelungsvorrichtung 25 kann beispielsweise als Rastmechanismus mit Rasthaken am einen Gehäuseteil und entsprechenden Öffnungen am anderen Gehäuseteil ausgebildet sein. Zum Lösen der Verbindung kann eine Grifffläche vorgesehen sein, die bei einer leichten Zugbewegung die Rastverbindung aufgrund einer gewissen Elastizität der verwendeten Materialien des Gehäuses löst.

Der Atemgasbefeuchter 1 kann über den Atemgaseinlass 2 und/oder den Atemgasauslass 4 an ein hier nicht dargestelltes Beatmungsgerät 50 angeschlossen werden. Hierfür können am Gehäuse des Atemgasbefeuchters 1 und/oder am Beatmungsgerät 50 ein oder mehrere hier nicht gezeigte Befestigungsvorrichtungen angeordnet sein. Die Befestigung kann mechanisch und/oder magnetisch und/oder elektrisch fixiert sein. Die Befestigung ist vorzugsweise reversibel ausgebildet, so dass eine einfache Befestigung und Lösung an das bzw. von dem Beatmungsgerät 50 vollzogen werden kann. Die Befestigungsvorrichtungen können beispielsweise als Haken am Beatmungsgerät 50 mit entsprechenden Gegenparten am Atemgasbefeuchter 1 ausgebildet sein oder vice versa. Die Haken können ausgebildet sein, um in den entsprechenden Gegenpart eingehängt, eingeclipt oder dergleichen zu werden. Der Atemgasbefeuchter 1 kann ferner mindestens eine Befestigungsrampe 26 umfassen. Die Befestigungsrampen 26 können als Rampen zum Abdrücken des Atemgasbefeuchters 1 vom Beatmungsgerät 50 dienen. Am Beatmungsgerät 50 kann mindestens eine gegenläufige Rampe angeordnet sein, die so ausgelegt ist, dass sie bei einer vertikalen Bewegung des Entriegelungsmechanismus zu einer horizontalen Bewegung (Loslösen) des Atemgasbefeuchters 1 führt.

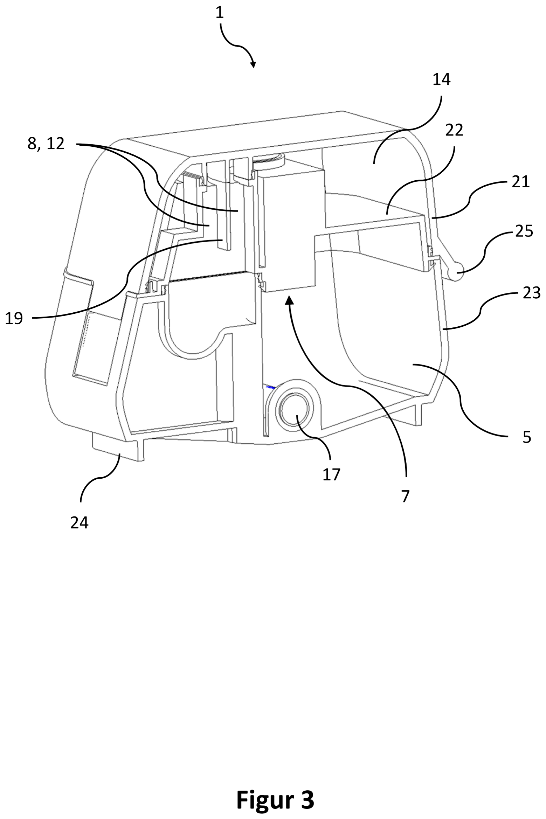

Fig. 3 zeigt einen perspektivischen Querschnitt eines Atemgasbefeuchters gemäß einer Ausführungsform der Erfindung. In diesem Beispiel ist gezeigt, dass das Mittelteil 22 zwischen dem Gehäuseoberteil 21 und dem Gehäuseunterteil 23 eingespannt sein kann. Das Mittelteil 22 ist derart atemgasdicht zwischen dem Gehäuseoberteil 21 und dem Gehäuseunterteil 23 eingespannt, dass das Atemgas entlang des Atemgaspfades geleitet werden kann. Der Atemgaspfad ist in diesem Beispiel durch das Gehäuseoberteil 21, das Gehäuseunterteil 23 und das Mittelteil 21 ausgebildet.

In diesem Ausführungsbeispiel weist der Kanal 8 zumindest abschnittweise ein Trennelement 19 auf. Das Trennelement 19 ist ausgebildet, um den Kanal 8 in seiner Längsrichtung betrachtet zumindest abschnittweise in mindestens zwei Teilkanäle zu unterteilen. Wie aus dieser Abbildung hervorgeht, kann der vierte Kanalabschnitt 12 in zwei Teilkanäle unterteilt sein. Das Trennelement 19 kann ausgebildet sein, um eine laminare Strömungsführung zu befördern und Turbulenzen im Kanal 8 zu unterbinden.

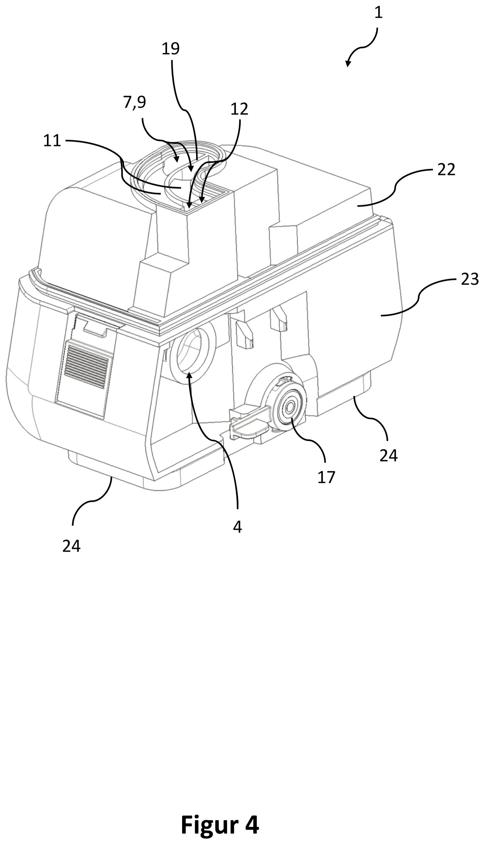

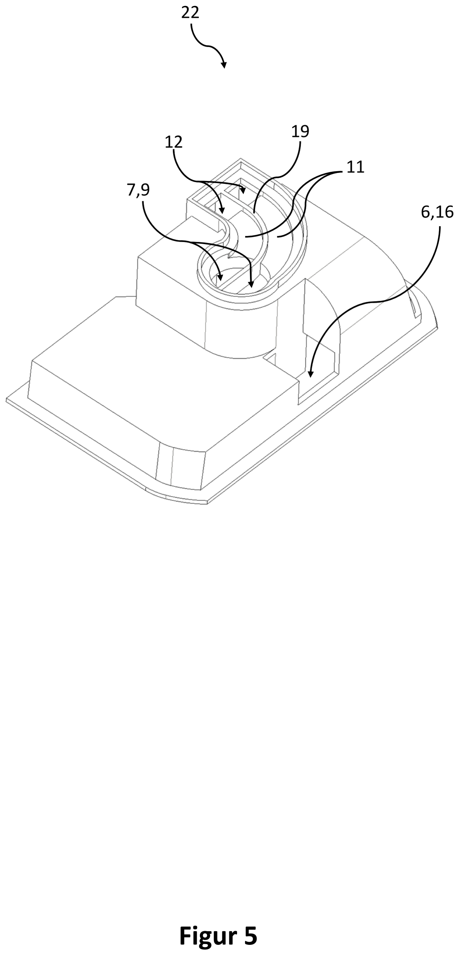

Fig. 4 zeigt eine perspektivische Außenansicht eines Atemgasbefeuchters ohne Darstellung des Gehäuseoberteils gemäß einer Ausführungsform der Erfindung. Fig. 5 zeigt eine perspektivische Außenansicht eines Mittelteils eines Atemgasbefeuchters gemäß einer Ausführungsform der Erfindung.

In diesem Beispiel ist der Wasserkammerauslass 7 und der erste Kanalabschnitt 9 mittig im Gerät angeordnet. Aus den Figuren 4 und 5 wird ersichtlich, dass das Trennelement 19 in manchen Ausführungsformen mehrere Teilabschnitte des Kanals 8 oder auch den ganzen Kanal 8 in zwei Teilkanäle unterteilen kann. Das Trennelement 19 unterteilt in diesem Beispiel zumindest den ersten Kanalabschnitt 9, den dritter Kanalabschnitt 11 und den vierten Kanalabschnitt 12 in zwei Teilkanäle. Das Trennelement 19 kann alternativ oder zusätzlich auch den Wasserkammerauslass 7 und/oder den zweiten Kanalabschnitt 10 in zwei Abschnitte bzw. Teilkanäle unterteilen. Die Anordnung mehrerer Trennelemente 19 zur Erzeugung weiterer Teilkanäle ist auch möglich (nicht gezeigt).

In dem Ausführungsbeispiel gemäß der Figuren 3 bis 5 ist darüber hinaus gezeigt, dass der Kanal 8 in seiner Querrichtung betrachtet zumindest bereichsweise gebogen ausgebildet sein kann. Es ist ersichtlich, dass beispielsweise der dritte Kanalabschnitt 11 in seiner Querrichtung betrachtet gebogen ausgebildet ist. Dadurch ist eine dreidimensionale Ausbildung des Kanals 8 und somit eine kompakte Bauform realisiert.

Aus Fig. 4 und 5 wird ersichtlich, dass der Kanal 8 erst durch eine Verbindung der Gehäuseteile vollständig ausgebildet ist. Der Kanal 8 kann beispielsweise zumindest bereichsweise zweiteilig ausgebildet sein, so dass der Kanal bei einer Trennung der Gehäuseteile zumindest bereichsweise geöffnet werden kann. Dies erleichtert eine Reinigung des Kanals 8.Der Kanal 8 kann zumindest abschnittsweise erst durch eine Verbindung des Gehäuseoberteils 21 mit dem Gehäuseunterteil 23 oder durch eine Verbindung des Gehäuseoberteils 21 mit dem Gehäuseunterteil 23 und dem Mittelteil 22 ausgebildet sein.

In den Abbildungen ist gezeigt, dass bereichsweise eine Hälfte des Kanals durch das Mittelteil 22 ausgebildet ist, in diesem Falle der dritte Kanalabschnitt 11. Die zweite Hälfte des Kanals 8 kann durch das hier nicht gezeigte Gehäuseoberteil 23 ausgebildet sein. Somit ist der Kanal 8 erst vollständig und/oder atemgasleitend ausgebildet, wenn die Gehäuseteile miteinander verbunden sind.

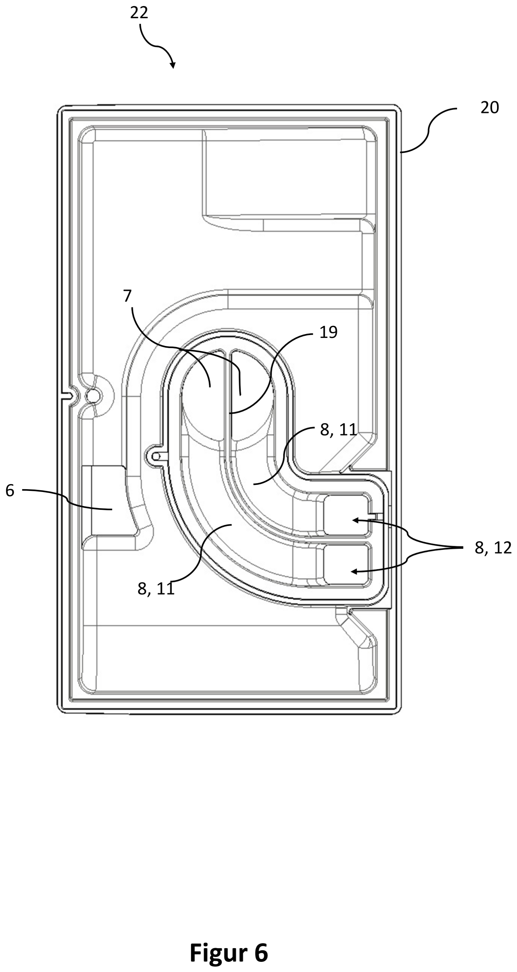

Fig. 6 zeigt eine Ansicht eines Mittelteils 22 von oben gemäß einer Ausführungsform der Erfindung. Aus Figur 6 wird ersichtlich, dass das Mittelteil 22 den Wasserkammereinlass 6 und den Wasserkammerauslass 7 zumindest teilweise ausbilden kann. Zudem kann das Mittelteil 22 zumindest teilweise zumindest bereichsweise den Kanal 8 ausbilden, wobei in dieser Abbildung der dritte Kanalabschnitt 11 zu sehen ist.

Der Wasserkammereinlass 6 kann als (einfache) Öffnung in dem Mittelteil 22 ausgebildet sein. Der Wasserkammereinlass 6 kann auch ein Volumen umfassen und kanalförmig ausgebildet sein und somit als Wasserkammereinlasskanal 16 im Mittelteil 22 ausgebildet sein. Das Atemgas kann entlang des Atemgaspfades 3 durch den Wasserkammereinlass 6 bzw. den Wasserkammereinlasskanal 16 in die Wasserkammer 5 gelangen. Der Wasserkammereinlass 6 bzw. der Wasserkammereinlasskanal 16 kann wie gezeigt benachbart zur Außenwand des Gehäuses 20 angeordnet sein.

Der Wasserkammerauslass 7 kann ebenfalls als (einfache) Öffnung in dem Mittelteil 22 ausgebildet sein. Der Kanal 8 kann an seinem einen Ende im Wasserkammerauslass 7 münden. Das Atemgas kann entlang des Atemgaspfades 3 durch den Wasserkammerauslass 7 aus der Wasserkammer 5 heraus in den Kanal 8 gelangen. Der Wasserkammerauslass 7 bzw. der Kanal 8 kann eingerichtet sein, das erwärmte und/oder befeuchtete Atemgas aus der Wasserkammer 5 heraus und zum hier nicht dargestellten Atemgasauslass 4 zu leiten.

Der Wasserkammerauslass 7 kann wie gezeigt mittig angeordnet sein. Sodann kann der Wasserkammerauslass 7 maximal beabstandet von den Außenwänden des Gehäuses 20 angeordnet sein. Dies bietet den Vorteil, dass die Flüssigkeit aus der Wasserkammer 5 nicht oder nur sehr erschwert in den Kanal 8 gelangen kann, wenn der Atemgasbefeuchter in eine Schräglage gerät. Der Kanal 8 kann ausgebildet sein, das Atemgas aus der Mitte heraus nach außen, also in Richtung der Gehäusewand 20 zu leiten. Der Kanal 8 mündet an seinem anderen Ende in den Atemgasauslass 4.

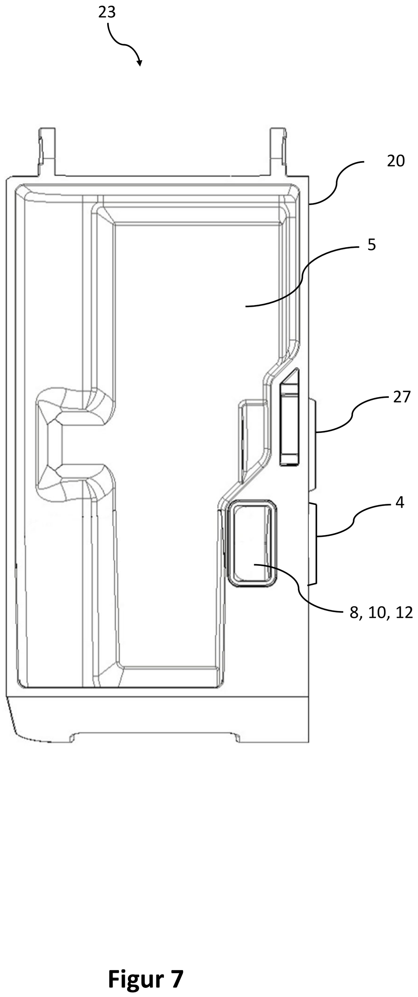

Fig. 7 zeigt eine Ansicht eines Gehäuseunterteils 23 von oben gemäß einer Ausführungsform der Erfindung. Aus Figur 7 wird ersichtlich, dass das Gehäuseunterteil 23 die Wasserkammer 5 umfassen kann. Zudem kann das Gehäuseunterteil 23 zumindest teilweise zumindest bereichsweise den Kanal 8 ausbilden. Aus dieser Figur wird ersichtlich, dass das Gehäuseunterteil 23 zumindest einen Teilabschnitt des Kanals 8 umfassen kann. Das Gehäuseunterteil 23 kann bevorzugt dienjenigen Kanalabschnitte 10,12 umfassen oder ausbilden, die benachbart zum Atemgasauslass 4 angeordnet sind bzw. in diesen münden.

Der Kanal 8 weist Kanalwand auf und ist über diese wasserdicht von der Wasserkammer getrennt 5 ausgebildet. Der Kanal 8 ist eingerichtet, um das erwärmte und/oder befeuchtete Atemgas aus der Wasserkammer 5 heraus in Richtung Atemgasauslass 4 zu leiten. Aus Fig. 7 wird ersichtlich, dass der Atemgasauslass 4 am Gehäuseunterteil 23 angeordnet ist bzw. durch dieses ausgebildet sein kann. Der Atemgasauslass 4 kann benachbart zur Heizelement-Aufnahme 27 angeordnet sein. Durch die Heizelement-Aufnahme 27 kann ein hier nicht gezeigtes Heizelement in die Wasserkammer 5 eingeführt bzw. gehaltert werden. Atemgasauslass 4 und Heizelement-Aufnahme 27 können bevorzugt an der gleichen Gehäusewand angeordnet sein.

Abschließend wird darauf hingewiesen, dass Begriffe wie "aufweisen", "umfassen", "einschließen", "mit" usw. keine anderen Elemente oder Schritte ausschließen und unbestimmte Artikel wie "ein" oder "eine" keine Vielzahl ausschließen.

Ferner wird darauf hingewiesen, dass Merkmale oder Schritte, die mit Verweis auf eine der vorstehenden Ausführungsformen beschrieben sind, auch in Kombination mit Merkmalen oder Schritten, die mit Verweis auf andere der vorstehenden Ausführungsformen beschrieben sind, verwendet werden können.

Bezugszeichen in den Ansprüchen sind nicht als Beschränkung des Umfangs des durch die Ansprüche definierten Gegenstands zu verstehen.

### Bezugszeichenliste

- 1: Atemgasbefeuchter
- 2: Atemgaseinlass
- 3: Atemgaspfad
- 4: Atemgasauslass
- 5: Wasserkammer
- 6: Wasserkammereinlass
- 7: Wasserkammerauslass
- 8: Kanal
- 9: Erster Kanalabschnitt
- 10: Zweiter Kanalabschnitt
- 11: Dritter Kanalabschnitt
- 12: Vierter Kanalabschnitt
- 14: Kammer
- 15: Strömungsleitelement
- 16: Wasserkammereinlasskanal
- 17: Heizelement
- 18: Flüssigkeit
- 19: Trennelement
- 20: Gehäuse
- 21: Gehäuseoberteil
- 22: Mittelteil
- 23: Gehäuseunterteil
- 24: Boden
- 25: Verriegelungsvorrichtung
- 26: Befestigungsrampe
- 27: Heizelement-Aufnahme
- 50: Beatmungsgerät
- 51: Erster Anschluss
- 52: Zweiter Anschluss
- 53: Schlauchanschluss
- 54: Leitung

## Patentansprüche

1. Atemgasbefeuchter (1) mit einem Gehäuse (20) umfassend ein Gehäuseoberteil (21) und ein Gehäuseunterteil (23) mit einer Wasserkammer (5) zum Befeuchten und/oder Erwärmen eines Atemgases, wobei der Atemgasbefeuchter (1) einen Atemgaseinlass (2) und einen Atemgasauslass (4) umfasst, wobei zwischen dem Atemgaseinlass (2) und dem Atemgasauslass (4) ein Atemgaspfad (3) zum Leiten des Atemgases vom Atemgaseinlass (2) zum Atemgasauslass (4) ausgebildet ist, der durch die Wasserkammer (5) geführt ist,
wobei der Atemgaspfad (3) einen wasserdichten Kanal (8) zum Leiten des befeuchteten und/oder angewärmten Atemgases umfasst, der an einem Ende (7) in die Wasserkammer (5) mündet und an einem anderen Ende in den Atemgasauslass (4) mündet,
wobei der Kanal (8) derart ausgebildet ist, dass der Atemgasauslass (4) am Gehäuseunterteil (23) angeordnet und/oder zumindest teilweise durch dieses ausgebildet ist.

2. Atemgasbefeuchter (1) nach Anspruch 1, wobei der Atemgasauslass (4) - wenn die Wasserkammer (5) bestimmungsgemäß mit einer Flüssigkeit zum Befeuchten des Atemgases befüllt ist - unterhalb der Flüssigkeitsoberfläche angeordnet ist.

3. Atemgasbefeuchter (1) nach einem der vorhergehenden Ansprüche, wobei der Kanal (8) zumindest abschnittweise durch das Gehäuseoberteil (21) und/oder das Gehäuseunterteil (23) ausgebildet ist oder durch diese verläuft.

4. Atemgasbefeuchter (1) nach einem der vorhergehenden Ansprüche, wobei der Atemgasbefeuchter (1) ein Mittelteil (22) aufweist, das atemgasdicht zwischen dem Gehäuseoberteil (21) und dem Gehäuseunterteil (23) angeordnet ist, wobei der Kanal (8) zumindest abschnittweise durch das Mittelteil (22) ausgebildet ist oder durch dieses verläuft.

5. Atemgasbefeuchter (1) nach einem der vorhergehenden Ansprüche, wobei der Kanal (8) zumindest abschnittsweise durch eine Verbindung des Gehäuseoberteils (21) mit dem Gehäuseunterteil (23) oder durch eine Verbindung des Gehäuseoberteils (21) mit dem Gehäuseunterteil (23) und dem Mittelteil (22) ausgebildet ist.

6. Atemgasbefeuchter (1) nach einem der vorhergehenden Ansprüche, wobei der Kanal (8) in seiner Längsrichtung betrachtet zumindest teilweise gewinkelt und/oder gebogen ausgebildet ist.

7. Atemgasbefeuchter (1) nach einem der vorhergehenden Ansprüche, wobei der Kanal (8) in seiner Längsrichtung betrachtet einen oder mehrere fluidisch miteinander in Verbindung stehende Kanalabschnitte (9, 10, 11, 12) umfasst, wobei der Kanal (8) einen ersten Kanalabschnitt (9) zur Verbindung mit einem Wasserkammerauslass (7), einen zweiten Kanalabschnitt (10) zur Verbindung mit dem Atemgasauslass (4) und mindestens einen den ersten Kanalabschnitt (9) mit dem zweiten Kanalabschnitt (10) verbindenden weiteren Kanalabschnitt (11,12) umfasst, wobei die Kanalabschnitte (9,10,11,12) ausgebildet sind, um mindestens eine Umlenkung des Atemgases zu bewirken.

8. Atemgasbefeuchter (1) nach Anspruch 7, wobei der erste Kanalabschnitt (9) so ausgebildet ist, dass er - wenn der Atemgasbefeuchter (1) bestimmungsgemäß auf dem Boden (24) steht und die Wasserkammer (5) mit einer Flüssigkeit zum Befeuchten des Atemgases befüllt ist - in vertikaler Richtung von der Flüssigkeitsoberfläche wegführt.

9. Atemgasbefeuchter (1) nach Anspruch 7 oder 8, wobei ein den ersten Kanalabschnitt (9) mit dem zweiten Kanalabschnitt (10) verbindender dritter Kanalabschnitt (11) so ausgebildet ist, dass er - wenn der Atemgasbefeuchter (1) bestimmungsgemäß auf dem Boden (24) steht - in vertikaler Richtung betrachtet oberhalb des Atemgasauslasses (4) angeordnet ist.

10. Atemgasbefeuchter (1) nach einem der vorhergehenden Ansprüche, wobei der Kanal (8) zumindest abschnittweise ein Trennelement (19) aufweist, das ausgebildet ist, um den Kanal (8) in seiner Längsrichtung betrachtet zumindest abschnittweise in mindestens zwei Teilkanäle zu unterteilen.

11. Atemgasbefeuchter (1) nach einem der vorhergehenden Ansprüche, wobei die Wasserkammer (5) einen Wasserkammereinlass (6) und einen Wasserkammerauslass (7) aufweist und der Atemgaspfad (3) durch den Wasserkammereinlass (6) in die Wasserkammer (5) hinein und durch den Wasserkammerauslass (7) aus der Wasserkammer (5) herausgeführt ist, wobei der Wasserkammereinlass (6) im Gehäuseoberteil (21) und/oder im Mittelteil (22) angeordnet ist, wobei der Wasserkammereinlass (6) als einfache Öffnung oder als Wasserkammereinlasskanal (16) ausgebildet ist.

12. Atemgasbefeuchter (1) nach Anspruch 11, wobei der Wasserkammerauslass (7) und/oder der erste Kanalabschnitt (9) in einer horizontalen Ebene betrachtet im Bereich der Mitte des Atemgasbefeuchters (1) angeordnet ist.

13. Atemgasbefeuchter (1) nach einem der vorhergehenden Ansprüche, wobei der Kanal (8) in seiner Querrichtung betrachtet zumindest teilweise gewinkelt und/oder gebogen ausgebildet ist, so dass der Atemgaspfad aus der Mitte des Atemgasbefeuchters (1) hin zum Gehäuse (20) und/oder zum Atemgasauslass (4) führt.

14. Atemgasbefeuchter (1) nach einem der vorhergehenden Ansprüche, wobei der Atemgasbefeuchter (1) eine Kammer (14) umfasst, die zwischen dem Atemgaseinlass (2) und dem Wasserkammereinlass (6,16) ausgebildet ist und Teil des Atemgaspfades (3) ist, wobei ein Querschnitt der Kammer (14) größer ist als ein Querschnitt des Atemgaseinlasses (2) und/oder ein Querschnitt des Wasserkammereinlasses (6) oder des Wasserkammereinlasskanals (16) und/oder des Kanals (8) ist.

15. Atemgasbefeuchter (1) nach einem der vorhergehenden Ansprüche, wobei die Kammer (14) und/oder der Kanal (8) und/oder die Wasserkammer (5) und/oder der Wasserkammereinlass (6) oder der Wasserkammereinlasskanal (16) akustische Impedanzen aufweisen und derart unterschiedlich dimensioniert sind, so dass ihre jeweiligen akustischen Impedanzen voneinander abweichen.

16. Beatmungsgerät (50) mit einem Atemgasweg, einem ersten Anschluss (51) und einem zweiten Anschluss (52), umfassend einen Atemgasbefeuchter (1) nach einem der vorhergehenden Ansprüche, wobei der Atemgasbefeuchter (1) über einen Atemgaseinlass (2) an den ersten Anschluss (51) und über einen Atemgasauslass (4) an den zweiten Anschluss (52) an den Atemgasweg das Beatmungsgerät (50) angeschlossen ist.
